# EUROPEAN PATENT SPECIFICATION

(11) **EP 4 146 305 B1**
(45) Date of publication and mention of the grant of the patent: **18.02.2026**
(21) Application number: 21720853.7
(22) Date of filing: 21.04.2021
(51) Int. Cl.: A61M 5/24

(54) **ADD-ON FOR MEDICAMENT DELIVERY DEVICE WITH MAGNETIC LOCK**
ZUSATZGERÄT FÜR MEDIKAMENTENABGABEVORRICHTUNG MIT MAGNETISCHEM SCHLOSS
APPOINT POUR DISPOSITIF D'ADMINISTRATION DE MÉDICAMENTS AVEC SERRURE MAGNETIQUE

(30) Priority: 08.05.2020 US 202063021683 P; 23.06.2020 EP 20181764
(43) Date of publication of application: 15.03.2023
(73) Proprietor: SHL Medical AG, 6300 Zug (CH)
(72) Inventor: SCHWARTZENTRUBER, Jared, New York, New York 10016 (US)
(86) International application number: PCT/IB2021/053272
(87) International publication number: WO 2021/224704

(56) References cited:
- WO-A1-2018/069022
- CN-A- 106 975 119
- CN-U- 205 339 743
- US-A1- 2013 110 046
- US-A1- 2015 273 138

## Description

### TECHNICAL FIELD

The invention concerns add-ons for medicament delivery devices, and particularly add-ons for medicament delivery devices that are locked together by magnetic locks.

### BACKGROUND

There has been a trend in recent years towards adding connectivity and sensors to medicament delivery devices such as autoinjectors. When adding connectivity and sensors to pre-existing medicament delivery devices, it is important that as few steps are added to the treatment regimen as possible, to minimise or avoid extra complexity for end users. For disposable devices, this implies that the embedded electronics should also be disposed of, but this is costly and is also environmentally suboptimal. The applicant has appreciated that improvements could be made in this area.

CN205339743 describes a liquid level monitoring alarm comprising first and second clamping parts. The first and second clamping parts incorporate a magnetic closure. CN106975119 describes a transfusion device with a magnetic closure. US2013/0110046 describes an apparatus for infusion equipment having a case with first and second portions. In one embodiment a magnetic fastener is provided for closing the case. WO2018/069022 describes a medicament delivery device with an auxiliary unit that may be attached to a mount by magnets.

### SUMMARY

The invention is defined by the appended claims, to which reference should now be made.

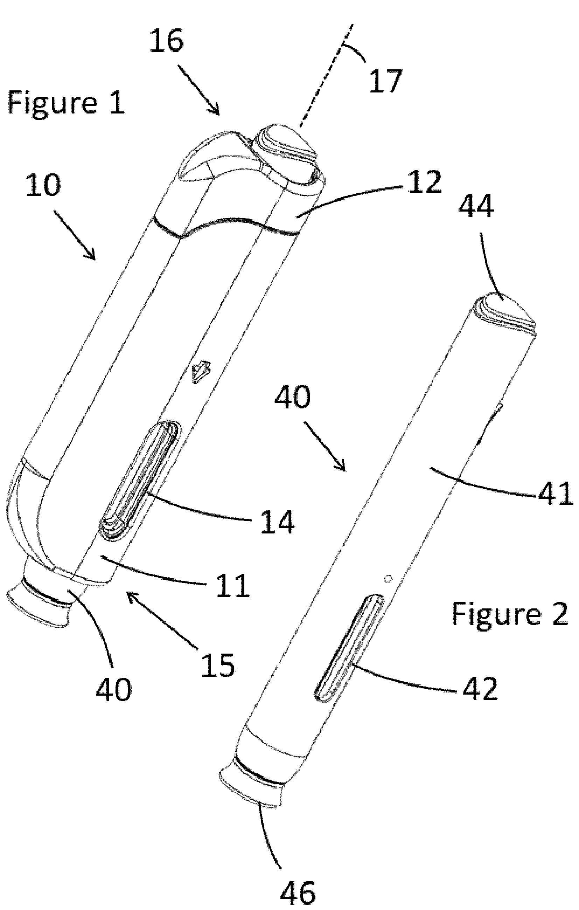

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which under use of the medicament delivery device is/are located furthest away from the dose delivery site. The distal end 16 of the add-on is shown in Figure 1, for example. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site. The proximal end 15 of the add-on is shown in Figure 1, for example.

Further, the terms "longitudinal", "longitudinally", "axially" and "axial" refer to a direction extending from the proximal end to the distal end and along the device or components thereof, typically in the direction of the longest extension of the device and/or component, namely the axis 17 as shown in Figure 1.

Similarly, the terms "transverse", "transversal" and "transversally" refer to a direction generally perpendicular to the longitudinal direction.

A first aspect of the invention concerns a medicament delivery device add-on comprising a first portion and a second portion. The first portion comprises at least one electronic component. The first portion is releasably attached to the second portion by a magnetic lock. This can make a connectivity add-on partly or entirely reusable. The magnetic lock can prevent the end user from removing the add-on from the drug delivery device, but can allow the manufacturer or a third party to recycle the device by unlocking it in the presence of a magnet. Recycling parts, particularly parts with electronic components, can significantly alter the economics (reduced cost) and environmental impact (reduced waste) of using add-ons. In addition, although there are many potential benefits to adding connectivity to the drug delivery device, it must be balanced with user experience, so as to not disrupt the user. Another benefit of this technology is that it can provide the same medicament delivery experience that the user is familiar with, but does not require the electronics to be disposed of. It can be useful when combined with any medicament delivery device, and can be particularly useful when combined with disposable medicament delivery devices, particularly single-use medicament delivery devices.

Preferably, the magnetic lock comprises a shim made of a magnetic metal. Preferably, the shim is flexible. Preferably, the shim is attached to one of the first portion and the second portion and is free to move relative to the other of the first portion and the second portion when a magnetic field is applied to the shim. Preferably, the end of the shim that is free to move is in a cut-out or a recess in the other of the first portion and the second portion until a magnetic field is applied to the shim. Preferably, the medicament delivery device add-on is configured such that the first portion is locked to the second portion in the absence of a magnetic field applied to the shim, and such that the first portion can be removed from the second portion when a magnetic field is applied to the shim.

Preferably, the electronic component comprises a sensor. Preferably, the sensor provides information about the state of the device. Preferably, the information comprises one or more of a use status and of a temperature.

A second aspect of the invention provides apparatus comprising a medicament delivery device and a medicament delivery device add-on as described above attached to the medicament delivery device. Preferably, the medicament delivery device is an autoinjector or a pen injector.

A third aspect of the invention concerns a method of removing a medicament delivery device from a medicament delivery device add-on, the method comprising the steps of applying a magnetic field to a magnetic shim in the medicament delivery device add-on to move the magnetic shim from a locked position to an unlocked position, then rotating a first portion of the medicament delivery device add-on relative to a second portion of the medicament delivery device add-on, then removing the first portion and the second portion from the medicament delivery device. This method could be carried out using the apparatus described above.

A fourth aspect of the invention concerns a medicament delivery apparatus comprising a medicament delivery device and an add-on, wherein the medicament delivery device is releasably attached to the add-on portion by a lock such as a magnetic lock. This can make it difficult or impossible for the end user to remove the medicament delivery device add-on from the add-on, but can allow someone with appropriate tools to easily remove the medicament delivery device add-on from the corresponding medicament delivery device.

Generally, all terms used in the claims are to be interpreted according to their ordinary meaning in the technical field, unless explicitly defined otherwise herein. All references to a/an/the element, apparatus, member, component, means, etc. are to be interpreted openly as referring to at least one instance of the element, apparatus, member component, means, etc., unless explicitly stated otherwise.

### BRIEF DESCRIPTION OF THE DRAWINGS

Embodiments of the invention will now be described by way of example only and with reference to the accompanying drawings, in which:
Figure 1 is a perspective view of an example medicament delivery device add-on and a corresponding autoinjector.
Figure 2 shows a perspective view of the autoinjector of Figure 1.
Figure 3 primarily shows a cross-section view of the medicament delivery add-on of Figure 1 with the first portion and second portion locked together.
Figure 4 primarily shows a cross-section view of the medicament delivery add-on of Figure 1 with the magnetic shim unlocked and the first portion and second portion rotated relative to one another.
Figure 5 shows a perspective view of part of the medicament delivery device add-on of Figure 1.
Figure 6 shows a cross-section view of part of the medicament delivery device add-on of Figure 1.

### DETAILED DESCRIPTION

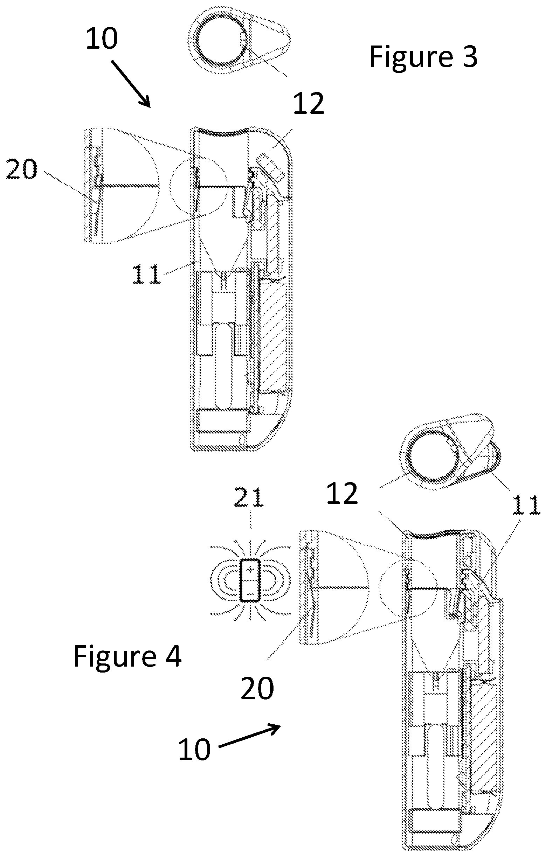

An example of the invention will now be described in more detail with reference to Figures 1 to 6. Figure 1 shows a medicament delivery device add-on 10 comprising a first portion 11 and a second portion 12. The first portion 11 comprises at least one electronic component (not shown). The first portion 11 is releasably attached to the second portion 12 by a magnetic lock, which in this example is a magnetic shim 20 (see Figures 3 to 5). An optional window 14 in the medicament delivery device add-on 10 can also be seen.

For context, Figure 1 shows the medicament delivery device add-on 10 attached to a medicament delivery device, in this case an autoinjector 40, although the medicament delivery device add-ons concepts described herein could also be used with other medicament delivery devices such as pen injectors. The autoinjector 40 is shown in Figure 2, and comprises in particular a housing 41, an optional window 42 in the housing 41, an activation button 44, and a cap 46. Other typical features of an autoinjector (not shown) include a medicament container comprising a medicament delivery member and a medicament, a powerpack for providing the force needed to inject the medicament and a medicament delivery member guard for protecting the medicament delivery member before and/or after injection.

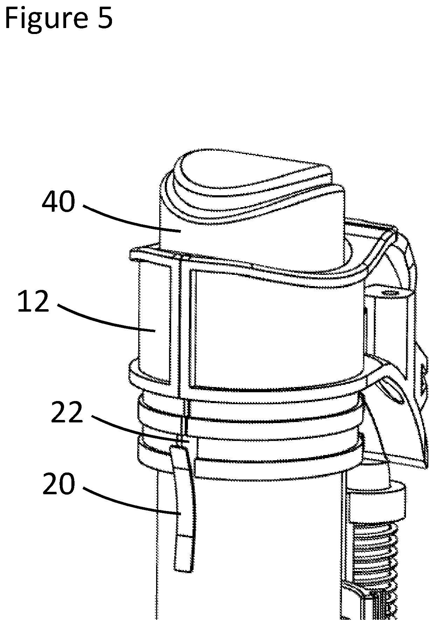

Figure 3 shows a cross-section of the medicament delivery device add-on 10 when the medicament delivery device add-on 10 is locked. As can be seen in the zoomed-in section, the magnetic shim 20 is straight. Although not directly visible in Figure 3, the magnetic shim 20 is fixed to the first portion 11 and is moveable relative to the second portion 12. A recess 22 in the second portion 12 (see Figure 5) accommodates the magnetic shim 20, stopping the second portion 12 from rotating relative to the first portion 11. When a magnet 21 is applied as shown in Figure 4, the magnetic shim 20 is bent, moving it out of the recess 22 and allowing the first portion 11 to rotate relative to the second portion 12.

Figure 5 shows part of the autoinjector 40 and part of the medicament delivery device add-on 10 (with most of the first portion 11 and part of the second portion 12 removed) to help provide context regarding the exact position of the magnetic shim 20.

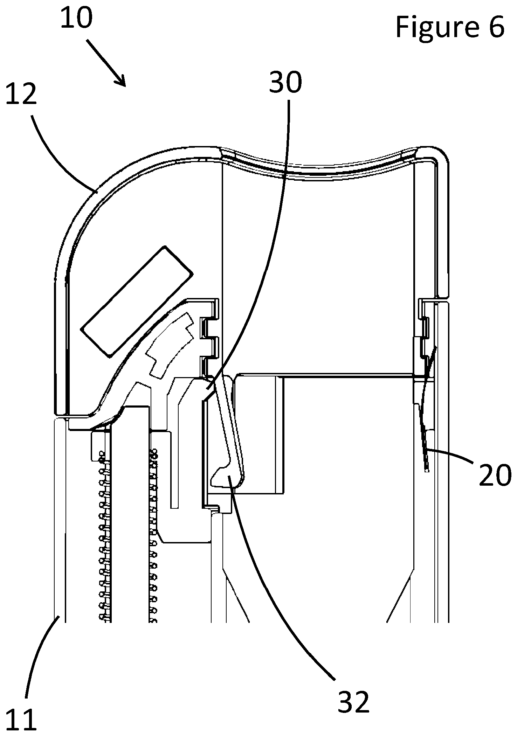

Figure 6 shows a cross-section of part of the medicament delivery device add-on 10. This view is shown primarily to highlight the two opposing hooks or protrusions, namely a hook or protrusion 30 on the first portion 11 and a hook or protrusion 32 on the second portion 12, which provide an example lock for locking the first portion to the second portion - whilst the magnetic shim stops the rotation of the first portion relative to the second portion, the hooks/protrusions 30, 32 are aligned axially, stopping the first portion from being fully removed from the second portion. When the first portion is able to rotate relative to the second portion (due to the shim being moved out of the way), the two hooks can be moved out of axial alignment, allowing them to pass one another and the first portion and second portion to be separated. When the device is being assembled, the first portion 11 and second portion 12 can be attached together, with the hook/protrusion 30 and the hook/protrusion 32 able to pass each other in the axial direction because the arms that the hooks/protrusions are attached to are flexible. Various alternative options and assembly methods could be used, with one or more hooks or protrusions instead inflexibly attached to the first portion and/or the second portion and with corresponding cut-out(s) or recess(es) to accommodate the one or more hooks or protrusions, for example with the assembly process being a reverse of the disassembly process described elsewhere herein. Providing flexible arms is not essential, although this can simplify assembly as only an axial motion is required (because the hooks/protrusions 30,32) can pass each other in the axial direction during assembly), rather than both axial and rotational motions.

The position after the first portion 11 and second portion 12 are attached together (but before an autoinjector is added) is shown in Figure 6. A medicament delivery device such as an autoinjector can subsequently be inserted into the add-on. The medicament delivery device stops the hooks/protrusions 30, 32 from passing each other in the axial direction, as it occupies the space that the flexible arm of hook/protrusion 32 would be in as it passes hook/protrusion 30. Although not shown in Figure 6, a recess or cut-out in the first portion 11 (for example in the flexible arm of the hook/protrusion 32) could be provided to allow the hook/protrusion 32 to move further out of the way of the medicament delivery device and further lock the first portion 11 and second portion 12 together.

In general, this idea allows a manufacturer or assembler to easily load and lock the underlying medicament delivery device to the add-on. The add-on can remain locked during interaction with the end-user, and can be unlocked during the recycling process when a magnetic field is in proximity to the locking mechanism.

To achieve this, the end user (e.g. the patient or the care giver) receives a medicament delivery device with the connected add-on rigidly attached. After medicament delivery, the combined unit is disposed of, including the medicament delivery device and the medicament delivery device add-on. If disposed of in a pre-determined container (like a sharps bin), the devices are recovered, and the add-on can be removed, sanitized and reused.

The methods of disassembly and use will now be discussed in more detail with reference to the example add-on above for context. As alluded to above, the first portion can be removed from the second portion by carrying out three steps. Firstly, a magnetic field is applied to the magnetic shim to move the magnetic shim from a locked position to an unlocked position (in the example above, this moves the magnetic shim 20 out of the recess 22). Secondly, the first portion and the second portion of the medicament delivery device add-on are rotated relative to each other (before rotation, the first portion and second portion are locked relative to each other in the axial direction, and after rotation the first portion and second portion are not locked relative to each other). Thirdly, the first portion and the second portion are removed from the medicament delivery device. Although not explicitly described above, the medicament delivery device (e.g. autoinjector 40) would typically comprise a protrusion, rib, recess and/or cut-out that can engage with a corresponding feature within the add-on. This could be an existing feature of a medicament delivery device (e.g. an anti-roll feature such as a recess or a protrusion), or could be added specifically for this purpose. This holds the medicament delivery device in place inside the medicament delivery device add-on. Other solutions such as a friction lock could alternatively be used to hold the medicament delivery device within the medicament delivery device add-on.

Although a particular shape of add-on is shown in the example above, other add-on shapes are also possible, as long as the add-on comprises two parts that can be separated from one another as described above. The invention is described with the electronic component in the first portion of the add-on, but the electronic component could alternatively be in the second portion, or two or more electronic components could be provided in the add-on (either in the first portion and/or the second portion). The concept described herein would also work without any electronic components in, although the value proposition for recycling add-ons with electronic components in will typically be better than that for add-ons without any electronic components.

The electronic component could comprise, for example, a sensor, a screen and/or an electronic communication component to communicate information (for example to communicate information from a sensor to another device such as a smartphone or a computer). In the case of a sensor, the sensor could provide information about the state of the device or the ambient temperature around the device, for example. In the case of information about the state of the device, the information could comprise the temperature and/or the use status of the add-on or of a corresponding medicament delivery device, for example.

The magnetic shim 20 is fixed to the first portion 11 and is moveable relative to the second portion 12 in the example described above. However, the magnetic shim could instead be fixed to the second portion 12 and moveable relative to the first portion 11, with other related features such as the recess 22 also switching portion accordingly. The magnetic shim is made of a magnetic metal or a magnetic alloy. The magnetic shim may be various shapes - in the example above, it is an elongated bar, but in general it may be a strip, a rod or another shape depending on the particular example. In addition, the shim is shown being bent by the magnet in a radial direction (perpendicular to the axis 17), but may be pulled in a different direction to unlock the add-on in other examples. The shim may also not bend, but rotate, translate, or some combination thereof to move the shim from a locked position to an unlocked position. The design of the recess 22 can also be altered depending for example on the shape of the first and second portions of the add-on and the shape of the shim, and could alternatively be a cut-out. Similarly, the hooks or protrusions 30 and 32 are provided just as one example, and various other concepts could be used for the axial locking element between the first and second portions.

The autoinjector 40 described above includes various features to provide an example, but in general none of the features shown are needed for the working of the invention. For example, a button 44 is provided, but is optional.

Various modifications to the embodiments described are possible and will occur to those skilled in the art without departing from the invention which is defined by the following claims.

## Claims

1. A medicament delivery device add-on (10) comprising
a first portion (11) and a second portion (12), the first portion (11) comprising at least one electronic component, **characterised by**
a magnetic lock comprising a magnetic shim (20), wherein the first portion (11) is releasably attached to the second portion (12) by the magnetic lock such that the first portion (11) is locked to the second portion (12) in the absence of a magnetic field applied to the magnetic shim (20), and such that the first portion (11) can be removed from the second portion (12) when a magnetic field is applied to the magnetic shim (20).

2. The medicament delivery device add-on (10) of claim 1, wherein the magnetic shim (20) is flexible and made of a magnetic metal.

3. The medicament delivery device add-on (10) of claim 1 or claim 2, wherein the magnetic shim (20) is attached to one of the first portion (11) and the second portion (12) and is free to move relative to the other of the first portion (11) and the second portion (20) when a magnetic field is applied to the shim.

4. The medicament delivery device add-on (10) of claim 3, wherein an end of the magnetic shim (20) that is free to move is in a cut-out or a recess (22) in the other of the first portion (11) and the second portion (12) until a magnetic field is applied to the magnetic shim (20).

5. The medicament delivery device add-on (10) of any one of claims 1 to 4, wherein the electronic component comprises a sensor.

6. The medicament delivery device add-on (10) of claim 5, wherein the sensor provides information about a state of the device.

7. The medicament delivery device add-on (10) of claim 6, wherein the information comprises one or more of a use status and of a temperature.

8. Apparatus comprising a medicament delivery device and a medicament delivery device add-on (10) according to any of claims 1 to 7 attached to the medicament delivery device.

9. Apparatus according to claim 8 wherein the medicament delivery device is an autoinjector (40) or a pen injector.

10. A method of removing a medicament delivery device from the medicament delivery device add-on according to any of claims 1 to 9 the method comprising the steps of applying a magnetic field to the magnetic shim to move the magnetic shim from a locked position to an unlocked position, then rotating the first portion of the medicament delivery device add-on relative to the second portion of the medicament delivery device add-on, then removing the first portion and the second portion from the medicament delivery device.

## Patentansprüche

1. Medikamentenabgabevorrichtungszusatz (10), umfassend
einen ersten Abschnitt (11) und einen zweiten Abschnitt (12), der erste Abschnitt (11) umfassend mindestens eine elektronische Komponente, **gekennzeichnet durch**
eine magnetische Verriegelung, umfassend eine magnetische Unterlegscheibe (20), wobei der erste Abschnitt (11) durch die magnetische Verriegelung derart lösbar an dem zweiten Abschnitt (12) befestigt ist, dass der erste Abschnitt (11) an dem zweiten Abschnitt (12) verriegelt ist, wenn kein magnetisches Feld vorhanden ist, das an der magnetischen Unterlegscheibe (20) anliegt, und dass der erste Abschnitt (11) von dem zweiten Abschnitt (12) entfernt werden kann, wenn ein magnetisches Feld an der magnetischen Unterlegscheibe (20) anliegt.

2. Medikamentenabgabevorrichtungszusatz (10) nach Anspruch 1, wobei die magnetische Unterlegscheibe (20) flexibel ist und aus einem magnetischen Metall hergestellt ist.

3. Medikamentenabgabevorrichtungszusatz (10) nach Anspruch 1 oder 2, wobei die magnetische Unterlegscheibe (20) an einem des ersten Abschnitts (11) und des zweiten Abschnitts (12) befestigt ist und sich relativ zu dem anderen des ersten Abschnitts (11) und des zweiten Abschnitts (20) frei bewegen kann, wenn ein magnetisches Feld an der Unterlegscheibe anliegt.

4. Medikamentenabgabevorrichtungszusatz (10) nach Anspruch 3, wobei sich ein Ende der magnetischen Unterlegscheibe (20), das sich frei bewegen kann, in einem Ausschnitt oder einer Aussparung (22) in dem anderen des ersten Abschnitts (11) und des zweiten Abschnitts (12) befindet, bis ein magnetisches Feld an der magnetischen Unterlegscheibe (20) anliegt.

5. Medikamentenabgabevorrichtungszusatz (10) nach einem der Ansprüche 1 bis 4, wobei die elektronische Komponente einen Sensor umfasst.

6. Medikamentenabgabevorrichtungszusatz (10) nach Anspruch 5, wobei der Sensor Informationen über einen Zustand der Vorrichtung bereitstellt.

7. Medikamentenabgabevorrichtungszusatz (10) nach Anspruch 6, wobei die Informationen eines oder mehrere umfassen von einem Verwendungsstatus und einer Temperatur.

8. Einrichtung, umfassend eine Medikamentenabgabevorrichtung und einen Medikamentenabgabevorrichtungszusatz (10) nach einem der Ansprüche 1 bis 7, der an der Medikamentenabgabevorrichtung befestigt ist.

9. Einrichtung nach Anspruch 8, wobei die Medikamentenabgabevorrichtung ein Autoinjektor (40) oder ein Pen-Injektor ist.

10. Verfahren zum Entfernen einer Medikamentenabgabevorrichtung von dem Medikamentenabgabevorrichtungszusatz nach einem der Ansprüche 1 bis 9, das Verfahren umfassend die Schritte des Anlegens eines magnetischen Felds an die magnetische Unterlegscheibe, um die magnetische Unterlegscheibe von einer verriegelten Position in eine entriegelte Position zu bewegen, dann eines Drehens des ersten Abschnitts des Medikamentenabgabevorrichtungszusatzes relativ zu dem zweiten Abschnitt des Medikamentenabgabevorrichtungszusatzes, dann des Entfernens des ersten Abschnitts und des zweiten Abschnitts von der Medikamentenabgabevorrichtung.

## Revendications

1. Accessoire de dispositif d'administration de médicament (10) comprenant
une première partie (11) et une seconde partie (12), la première partie (11) comprenant au moins un composant électronique, **caractérisé par**
une serrure magnétique comprenant une cale magnétique (20), dans lequel la première partie (11) est attachée de manière amovible à la seconde partie (12) par la serrure magnétique, de telle sorte que la première partie (11) est verrouillée à la seconde partie (12) en l'absence d'un champ magnétique appliqué à la cale magnétique (20), et de telle sorte que la première partie (11) peut être retirée de la seconde partie (12) lorsqu'un champ magnétique est appliqué à la cale magnétique (20).

2. Accessoire de dispositif d'administration de médicament (10) selon la revendication 1, dans lequel la cale magnétique (20) est flexible et faite d'un métal magnétique.

3. Accessoire de dispositif d'administration de médicament (10) selon la revendication 1 ou la revendication 2, dans lequel la cale magnétique (20) est attachée à l'une de la première partie (11) et de la seconde partie (12) et est libre de se déplacer par rapport à l'autre parmi la première partie (11) et la seconde partie (20) lorsqu'un champ magnétique est appliqué à la cale.

4. Accessoire de dispositif d'administration de médicament (10) selon la revendication 3, dans lequel une extrémité de la cale magnétique (20) qui est libre de se déplacer se trouve dans une découpe ou un renfoncement (22) dans l'autre parmi la première partie (11) et la seconde partie (12) jusqu'à ce qu'un champ magnétique soit appliqué à la cale magnétique (20).

5. Accessoire de dispositif d'administration de médicament (10) selon l'une quelconque des revendications 1 à 4, dans lequel le composant électronique comprend un capteur.

6. Accessoire de dispositif d'administration de médicament (10) selon la revendication 5, dans lequel le capteur fournit des informations concernant un état du dispositif.

7. Accessoire de dispositif d'administration de médicament (10) selon la revendication 6, dans lequel les informations comprennent un ou plusieurs parmi un état d'utilisation et une température.

8. Appareil comprenant un dispositif d'administration de médicament et un accessoire de dispositif d'administration de médicament (10) selon l'une quelconque des revendications 1 à 7, attaché au dispositif d'administration de médicament.

9. Appareil selon la revendication 8, dans lequel le dispositif d'administration de médicament est un auto-injecteur (40) ou un stylo injecteur.

10. Procédé permettant de retirer un dispositif d'administration de médicament de l'accessoire de dispositif d'administration de médicament selon l'une quelconque des revendications 1 à 9, le procédé comprenant les étapes consistant à appliquer un champ magnétique à la cale magnétique pour déplacer la cale magnétique d'une position verrouillée à une position déverrouillée, puis à faire tourner la première partie de l'accessoire de dispositif d'administration de médicament par rapport à la seconde partie de l'accessoire de dispositif d'administration de médicament, puis à retirer la première partie et la seconde partie du dispositif d'administration de médicament.
